# EUROPÄISCHE PATENTSCHRIFT

(11) **EP 2 675 305 B1**
(45) Veröffentlichungstag und Bekanntmachung des Hinweises auf die Patenterteilung: **28.11.2018**
(21) Anmeldenummer: 12717171.8
(22) Anmeldetag: 17.02.2012
(51) Int. Cl.: A24F 47/00

(54) **RAUCHFREIE ZIGARETTE, ZIGARRE ODER PFEIFE**
SMOKE-FREE CIGARETTE, CIGAR OR PIPE
CIGARETTE, CIGARE OU PIPE SANS FUMÉE

(30) Priorität: 18.02.2011 DE 102011011676
(43) Veröffentlichungstag der Anmeldung: 25.12.2013
(73) Patentinhaber: Surflay Nanotec GmbH, 12489 Berlin (DE); Severus Patent AG, 6343 Rotkreuz (CH)
(72) Erfinder: DÄHNE, Lars, 15366 Dahlwitz Hoppegarte (DE); EGRI, Gabriella, 12526 Berlin (DE); TRESCHER, Heinrich, 67549 Worms (DE)
(74) Vertreter: Weigel, Matthias
(86) Internationale Anmeldenummer: PCT/EP2012/000727
(87) Internationale Veröffentlichungsnummer: WO 2012/110258

(56) Entgegenhaltungen:
- EP-A1- 0 241 698
- EP-A2- 0 277 519
- WO-A1-2011/083168
- DE-A1-102005 054 255

## Beschreibung

Die Erfindung betrifft eine rauchfreie Zigarette, Zigarre oder Pfeife mit mindestens einem Depot für die Speicherung und definierte, durch externe Wärmezufuhr bewirkte Freisetzung von Nikotin und/oder mindestens einer nikotinhaltigen Verbindung an einen durch das Depot zu leitenden Luftstrom.

Bereits seit geraumer Zeit werden intensive Versuche unternommen, sogenannte rauchfreie Zigaretten, Zigarren oder Pfeifen zu entwickeln, also Einrichtungen, die zur gezielten und definierten Freisetzung von Nikotin und/oder nikotinhaltigen Verbindungen dienen, ohne dass hierbei Tabak unter Rauchentwicklung verbrannt wird. Unter dem Begriff "nikotinhaltige Verbindung" werden in der vorliegenden Anmeldung insbesondere Nikotinsalze, Nikotinderivate sowie nikotinhaltige Tabakinhaltsstoffe verstanden.

Problematisch bei den derzeit auf dem Markt erhältlichen Produkten ist, dass insbesondere eine definierte Freisetzung des Nikotins bzw. der nikotinhaltigen Verbindung häufig nicht gewährleistet ist.

So sind elektronische Zigaretten bekannt, bei denen der angesaugte Luftstrom vor dem Durchströmen des Depots zur Freisetzung des Nikotins bzw. der nikotinhaltigen Verbindung auf eine ausreichend hohe Temperatur erwärmt werden muss. Nachteilig bei dieser bekannten Lösung ist, dass die Trägheit des gesamten Systems, also die Reaktionszeit vom ersten Ansaugen des Benutzers bis eine ausreichend hohe Temperatur im Luftstrom bereitgestellt werden kann, um das Nikotin bzw. die nikotinhaltige Verbindung freizusetzen, zu lange ist, um eine ausreichende und definierte Freisetzung des Nikotins bzw. der nikotinhaltigen Verbindung aus dem Depot zu gewährleisten. Ferner ist die Energiemenge, die notwendig ist, um den Luftstrom auf eine ausreichend hohe Temperatur zu erwärmen, üblicherweise liegt diese bei etwa 80°C, so hoch, dass die erforderlichen Energiespeicher, wie Akkumulatoren, schnell entleert sind und aufgrund der hohen Energiemengen bereits nach wenigen Ladevorgängen nicht mehr ordnungsgemäß arbeiten.

Aus der WO 2011/083168 ist eine rauchfreie Zigarette bekannt, bei der Nikotin oder nikotinhaltige Verbindungen in einem Depot gespeichert sind, aus dem das Niktoin oder die niktoinhaltige Substanz durch Wärmeeinbrinungen gezielt freigesetzt werden kann. Das bekannte Depot weist hierzu Hybridstruktur aus einem offenporigen makroporösen Stützgerüst auf, durch das der Luftstrom strömen kann, wobei das Stützgerüst vorgesehenen Nanoporen zur Speicherung und Freisetzung des Nikotins und/oder der nikotinhaltigen Verbindung aufweist. Das Stützgerüst ist hierbei aus dauerhaft miteinander verbundenen Partikeln oder Fasern gebildet, während die Nanoporen in nanoporösen Partikeln ausgebildet sind, welche im Stützgerüst immobilisiert gehalten sind.

Aus dem Dokument DE 10 2005054 255 A1 ist eine rauchfreie Zigarette gemäß dem Oberbegriff des Anspruchs 1 bekannt. Ausgehend von diesem Stand der Technik ist es Aufgabe der Erfindung eine rauchfreie Zigarette, Zigarre oder Pfeife bereitzustellen, bei der die Freisetzung des Nikotins und/oder der mindestens einen nikotinhaltigen Verbindung aus dem Depot sehr gezielt erfolgt.

Erfindungsgemäß wird diese Aufgabe durch eine rauchfreie Zigarette, Zigarre oder Pfeife mit den Merkmalen des Anspruchs 1 gelöst.

Bei der erfindungsgemäßen rauchfreien Zigarette ist das Depot der Zigarette mit einem Wärmeübertragungsabschnitt versehen. Mit Hilfe des Wärmeübertragungsabschnittes wird gezielt Wärme von außen in das Innere des Depots geleitet, um eine definierte und gleichmäßige Freisetzung des Nikotins bzw. der nikotinhaltigen Verbindung aus dem Depot zu bewirken. Durch das Vorsehen des Wärmeübertragungsabschnittes wird auch erreicht, dass die für die Freisetzung des Nikotins bzw. der nikotinhaltigen Verbindung erforderliche Wärmemenge verglichen mit den aus dem Stand der Technik bekannten Lösungen sehr gering ist. So erfolgt die Wärmeübertragung durch Wärmeleitung oder durch Wärmestrahlung unmittelbar von außen in das Depot, wodurch auftretende Wärmeverluste minimiert sind. Der Wärmeübertragungsabschnitt ist dabei vorzugsweise so konzipiert, dass die an den Wärmeübertragungsabschnitt zugeführte Wärme gleichmäßig im Depot verteilt wird. Als Material wird ein Material verwendet, dessen Wärmeleitfähigkeit in einem Bereich von 30 bis 450 W/(m·K) liegt. Vorzugsweise wird ein Material verwendet, dessen Wärmeleitfähigkeit in einem Bereich von 85 bis 300 W/(m·K), besonders bevorzugt in einem Bereich von 185 bis 250 W/(m·K) liegt. Als Material für den die Wärme übertragenden Abschnitt eignen sich insbesondere Aluminium, Kupfer, Eisen oder Legierungen aus diesen Bestandteilen.

Bei der Suche nach einem geeigneten Material für das Depot selbst wurde überraschend gefunden, dass sich Hybridstrukturen, also Strukturen aus unterschiedlichen Materialien, die eine hierarchische Porosität aufweisen, besonders gut eignen, um das Nikotin und/oder nikotinhaltige Verbindungen sowie gegebenenfalls Aromastoffe und Geschmacksstoffe einerseits gut zu speichern und andererseits bei entsprechender Wärmezufuhr in ausreichenden Mengen freizusetzen. Ferner wirkt die Hybridstruktur darüber hinaus als eine Art Filter.

Bei der erfindungsgemäßen rauchfreien Zigarette, Zigarre oder Pfeife weist das Depot eine Hybridstruktur aus einem offenporigen makroporösen Stützgerüst auf, durch das der Luftstrom strömen kann, während im Stützgerüst vorgesehenen Nanoporen zur Speicherung und Freisetzung des Nikotins und/oder der nikotinhaltigen Verbindungen und gegebenenfalls weiterer Aroma- und Geschmacksstoffe dienen.

Versuche zur Langzeitstabilität von Nikotin im beschriebenen Depot im Vergleich zu üblicherweise verwendeten Trägermaterialien, wie Acetatfasern, ergaben eine deutlich verringerte Gelb- bzw. Braunfärbung. Das in nanoporösen Partikeln vorliegende Nikotin ist im Vergleich zu Nikotin in einem saugfähigen Träger, beispielsweise Acetatfasern, stabiler gegenüber einer Oxidation an der Luft. Das ist bemerkenswert, da die von den nanoporösen Partikeln bereitgestellte Kontaktfläche zur Luft sehr groß ist. Es wird vermutet, ohne sich darauf einschränken zu wollen, dass sich die Oxidationsempfindlichkeit von Nikotin nach Adsorption an der Oberfläche, beispielsweise die innere und äußere Oberfläche von Silika-Partikeln, des nanoporösen Materials deutlich verringert.

Vorteilhafte Weiterbildungen der Erfindung ergeben sich aus der nachfolgenden Beschreibung, den Unteransprüchen sowie der Zeichnung.

Bei einer besonders bevorzugten Ausführungsform der erfindungsgemäßen rauchfreien Zigarette weist das Depot eine das Depot zumindest teilweise ummantelnde Hülse auf, welche für eine gezielte Wärmezufuhr zumindest abschnittsweise aus einem Material mit hoher Wärmeleitfähigkeit gefertigt ist. Die Hülse, die üblicherweise an beiden Enden zumindest teilweise geöffnet ist, dient dabei als Schutz des Depots an sich. Das Depot ist hierbei entweder fest in die Hülse eingefügt oder aber die Hülse ist nachträglich auf das Depot aufgeschoben. Auf alle Fälle muss eine gute externe Wärmeübertragung durch die Hülse auf das Depot sichergestellt sein. Die Hülse hat vorzugsweise eine hohlzylindrische runde Innenform, während die Außenseite gegebenenfalls rund, oval oder auch mehreckig ausgebildet sein kann. Auch ist eine gestufte Ausbildung der Außenmantelfläche der Hülse möglich. Besonders von Vorteil ist es, wenn der wärmeübertragende Abschnitt der Hülse sich an der mit dem Depot in Kontakt stehenden Innenwand über die gesamte axiale Länge des Depots erstreckt, während die Außenseite der Hülse nur in dem Bereich aus dem wärmeleitenden Material gefertigt ist, welches mit der externen Wärmequelle zusammenwirkt.

Bei einer weiteren bevorzugten Ausführungsform befindet sich die Wärmeübergangsfläche im Inneren des Depots, wobei die äußere Hülse aus einem möglichst wärmeisolierendem Material gefertigt ist. Das Depot hat in diesem Fall vorteilhafterweise eine Einbuchtung, die unterschiedliche geometrische Form habe kann wie z.B. zylindrisch aber auch schlitzförmig, rechteckig oder kugelförmig. In diese Einbuchtung ragt ein Heizstab aus einem Material mit hoher Wärmeleitfähigkeit, der durch externe Energiezufuhr, wie beispielsweise Strom erhitzt wird. Für den die Wärme übertragenden Abschnitt der Hülse bzw. des Stabes wird vorgeschlagen, das Material mit vergleichsweise hoher Wärmeleitfähigkeit zu verwenden. Sofern die Hülse nur teilweise aus diesem Material gefertigt ist, kann der nicht für die Wärmeübertragung gedachte Abschnitt auch aus einem isolierenden Material gefertigt sein, um eine ungewollte Wärmeableitung zu vermeiden.

Das Stützgerüst besteht aus dauerhaft miteinander verbundenen Partikeln oder Fasern, während die Nanoporen in nanoporösen Partikeln ausgebildet sind, welche im Stützgerüst immobilisiert gehalten sind. Bei dieser Lösung bestehen die nanoporösen Partikel vorzugsweise aus einem anderen Material als das Stützgerüst.

Gemäß einer bevorzugten Ausführungsform werden die nanoporösen Partikel zusammen mit dem partikulären Material (Mikropartikel, Fasern oder dergleichen) gemischt und dann das partikuläre Material miteinander zur Bildung eines offenporigen Stützgerüsts verbunden, wobei dadurch gleichzeitig die nanoporösen Partikel im oder am Stützgerüst immobilisiert werden.

Besonders bevorzugt weisen die nanoporösen Partikel im Vergleich zum Material, aus dem das Stützgerüst besteht, oder im Vergleich zu den Partikeln, aus denen das Stützgerüst gebildet wird, eine höhere Schmelztemperatur auf. Dadurch wird gewährleistet, dass bei einer thermischen Behandlung zur Verfestigung des Stützgerüsts die nanoporösen Partikel nicht schmelzen oder deren Poren verkleben. Die nanoporösen Partikel können somit in die Oberfläche der Stützgerüstpartikel beim Erweichen "eingebettet" werden.

Ein besonderer Vorteil der an der Oberfläche eines makroporösen Stützgerüsts dauerhaft durch Sintern befestigten nanoporösen Partikeln mit hoher spezifischer Oberfläche und Affinität für Wirk- und Aromastoffe und deren Verwendung in einem Depot zur Substanz-Freisetzung in durchströmende Luft besteht darin, dass lediglich die jeweils zuvor gebundenen Substanzen freigesetzt werden. Im Luftstrom, welcher das Depot verlässt, treten neben den gasförmig vorliegenden Wirk-und/oder Aromastoffen vorzugsweise keine Flüssigkeitströpfchen irgendwelcher Hilfssubstanzen oder größere Mengen an gesundheitsgefährdenden Partikel auf. Die Fixierung der nanoporösen Partikel am makroporösen Stützgerüst verhindert, dass die nanoporösen Partikel vom Luftstrom mitgerissen werden können.

Alternativ oder auch ergänzend hierzu kann das Stützgerüst auch im wesentlichen aus einem anorganischen Material, wie Glas, Silikaten oder Alumosilikaten, gebildet sein, während die Nanoporen im Material des Stützgerüstes selbst ausgebildet sind.

Die Menge und die Größe der nanoporösen Partikel sind so gewählt, dass die Makroporen einerseits nicht verstopft werden und andererseits genügend Wirkstoff im Inneren der Partikel adsorbiert werden kann. Das Nikotin bzw. die nikotinhaltige Verbindung liegen im Inneren der nanoporösen Partikeln beispielsweise adsorbiert vor. Insbesondere können die Nikotin bzw. die nikotinhaltige Verbindung sowie eventuell eingebrachte Aroma- und Geruchsstoffe direkt in den Nanoporen vorliegen, d.h. ohne dass sie in einem Lösungsmittel gelöst sind.

Das Stützgerüst ist vorzugsweise aus Partikeln, Fasern, partikulärem Material, oder Gemischen aus diesen Materialien aufgebaut. Das partikuläre Material oder Materialgemisch kann beispielsweise gesintert, angeschmolzen, geklebt oder anderweitig untereinander verbunden werden, so dass ein mechanisch stabiles offenporiges Stützgerüst entsteht.

Die Fixierung der nanoporösen Partikeln an der Oberfläche des beispielsweise beim Sintern gebildeten makroporösen Stützgerüsts kann so erfolgen, dass die Nanoporen des fixierten Partikels auf seiner von der Oberfläche des Stützgerüsts abgewandten Seite nicht verschlossen werden. Auch beim Befüllen des Depots wird das Verschließen dieser Poren vermieden.

Die Freisetzung der in der Hybridstruktur gebunden oder adsorbiert vorliegenden Substanzen in den Luftstrom erfolgt bevorzugt durch Konvektion und/oder Diffusion. Die bevorzugten strukturellen Besonderheiten des Depots, beispielsweise des Stützgerüstes, können sich aus den für die jeweilige Anwendung jeweils bevorzugten Freisetzungsraten der im Depot vorliegenden Substanzen ergeben.

Des weiteren liegt es im Umfang der Erfindung, das Depot so auszubilden, dass die Hybridstruktur aus einem offenporigen makroporösen Stützgerüst mit Kanälen gebildet ist, durch die der Luftstrom leicht hindurch strömen kann, während die Nanoporen den freizusetzenden Wirkstoff enthalten. Die Kanäle weisen beispielsweise eine mittlere Weite auf, die insgesamt ein Durchströmen des Luftstromes gestatten. Beispielsweise kann die Kanalweite in einem Bereich einiger hundert Mikrometer liegen, beispielsweise zwischen 100µm und 800µm. Es versteht sich jedoch von selbst, dass diese Angabe sich auf eine mittlere Kanalweite bezieht, da die Kanäle eine unregelmäßige Form und auch eine Kanalweitenverteilung aufweisen können.

Die zur Bildung des Stützgerüsts genutzten Mikropartikel des Depots weisen Größen von 200 bis 1000 µm auf. Die nanoporösen Partikel werden an der Oberfläche des Stützgerüstes beispielsweise durch Sintern dauerhaft fixiert. Dabei ist es denkbar, dass das offenporige Stützgerüst Makroporen aufweist, die größer sind, als die nanoporösen Partikel, welche zur Aufnahme des Nikotins bzw. der nikotinhaltigen Verbindung dienen. Die Größe der im Stützgerüst vorliegenden Makroporen liegt dann typischerweise in einem Bereich zwischen 10 µm und 800 µm, beispielsweise zwischen 20 µm und 700 µm, insbesondere zwischen 25 µm und 600 µmDie genannten nanoporösen Partikel weisen vorzugsweise mittlere Partikeldurch-messer von 5 µm bis 500 µm, besonders bevorzugt Durchmesser zwischen 10 µm bis 300 µm und insbesondere bevorzugt Durchmesser zwischen 20 µm und 150 µm auf. Ihre Nanoporen liegen typischerweise in einem Bereich zwischen 1 nm bis 900 nm. So liegen beispielsweise ihre Porendurchmesser in einem Bereich von 3 nm bis 700 nm, vorzugsweise in einem Bereich zwischen 6 nm bis 500 nm.

In verschiedenen Ausführungsformen dienen partikel- oder faserförmige Sintermaterialien aus anorganischen Materialien, wie beispielsweise Silikate oder Alumosilikate, zur Bildung des makroporösen Stützgerüsts des Depots. Ebenso können organische Polymere für das makroporöse Stützgerüst verwendet werden, wie beispielsweise Polyethylen oder Polypropylen, Polyvinylchlorid, Polycarbonat, Polyurethan, Polyester und weitere Materialien. Aufgrund der Verwendung des Depots für Zigaretten, Zigarren oder Pfeifen sollte darauf geachtet werden, dass keine Weichmacher in den Polymeren enthalten sind. Im Fall von partikulären Sintermaterialien sollte deren Partikelgröße so angepasst sein, dass bei der gewünschten Länge des Depots die Luft leicht hindurch strömen kann.

Das Depot kann auch durch ein Gemisch aus unterschiedlich großen Stützpartikeln oder Fasern bzw. aus Partikeln die aus unterschiedlichen Materialien bestehen, gebildet werden. Ebenso können verschiedene nanoporöse Partikel gleichzeitig in dem Stützgerüst eingebettet werden, die sich in ihrer Größe und/oder Form und/oder Porengröße und/oder Material und/oder Affinität zu dem Wirkstoff unterscheiden. Die zur Herstellung des Depots verwendeten Partikel können ebenso ein Gemisch unterschiedlich beladener oder mit unterschiedlichen Substanzen beladener poröser Partikel sein.

Die Größe und die Form des Depots werden an die jeweilige Anwendung angepasst. Sie ist insbesondere adaptiert an die geometrischen Gegebenheiten der Zigarette, Zigarre bzw. Pfeife. Beispielsweise kann das Depot als Einweg-Depot für die Unterbringung im Kopf oder Holm einer konventionellen Tabakspfeife gestaltet werden oder die Größe, Form und Farbe eines Zigarettenfilters aufweisen oder zum Einsatz in einem Imitat einer Zigarre oder eines Zigarillos hergerichtet sein.

Mit der Anpassung wird eine gute Zugänglichkeit der Nanoporen für den jeweils anströmenden Luftstrom und die gleichmäßige Verteilung der im Depot gespeicherten Substanzen erreicht. Dabei kann die Form des Depots und/oder der Kartusche vorteilhafterweise zylindrisch aber auch eckig sein, wie beispielsweise kubisch oder quaderförmig. Gemäß einer oder mehrere Ausführungsformen weist ein austauschbarer Filterstick für eine rauchfreie Zigarette das Depot auf.

Je nach Verwendungszweck wird die Länge und die Breite bzw. der Durchmesser des makroporösen Stützgerüstes in Abhängigkeit von der Flüchtigkeit des Wirkstoffes, der Temperatur des ankommenden Luftstromes, der gewünschten Konzentration des Nikotins des das Depot verlassenden Luftstromes, der Beladungsmenge, dem Strömungswiderstand, sowie der dafür zu immobilisierenden Menge nanoporöser Partikeln geeignet gewählt werden.

Überraschenderweise eignen sich anorganische Adsorbermaterialien mit einer möglichst großen inneren Oberfläche gut als nanoporöse Depotmaterialien für das Nikotin bzw. die nikotinhaltigen Verbindungen sowie gegebenenfalls gespeicherte Wirkstoffe und Aromastoffe. Die innere Oberfläche der Adsorbermaterialien wird von den Wänden der Poren gebildet, so dass eine große Kontaktfläche für porengängige Substanzen zur Verfügung steht. Neben porösen Alumosilikaten eignen sich nanoporöse Silikate besonders gut zur Aufnahme von Nikotin und Aromastoffen.

Diese Adsorbermaterialien weisen ein offenporiges Netz miteinander verbundener Röhren auf. Sie sind mit definierten mittleren Porenweiten, zumeist von 3 nm, 7 nm, 12 nm, 30 nm und 100 nm Porendurchmesser als chromatographische Trägermaterialien kommerziell erhältlich. Möglich sind aber auch breitere Porenverteilungen im Nanometerbereich, wie z.B. von 3 nm bis 700 nm.

Die Partikelgröße geeigneter nanoporöser Materialien liegt beispielsweise bei 10 µm bis 1 mm, insbesondere von 50 µm bis 700 µm. Die nanoporösen Materialien können eine sphärische Form aufweisen. Gemäß einer besonders bevorzugten Ausführungsform werden zur Einbettung irregulär gebrochene Materialien und damit irregulär geformte Partikel verwendet.

Bei einer Ausführungsform weist die rauchfreie Zigarette, Zigarre oder Pfeife zwei oder mehr Depots auf, die miteinander kombiniert werden und in denen jeweils unterschiedliche Substanzen adsorbiert sind. Dabei ist wenigstens eines der Depots mit einem Wärmeübertragungsabschnitt versehen. Beispielsweise kann eine der Substanzen zur teilweisen oder vollständigen Modifikation, beispielsweise chemischen Umwandlung, der anderen Substanz dienen. So wird beispielweise bei Verwendung von Nikotin, dem Nikotin enthaltenen Depot ein weiteres Depot vorgeordnet, in dem eine Säure gespeichert ist, welche das Nikotin zumindest teilweise in ein Nikotinsalz überführt, um die Verträglichkeit des inhalierten Nikotins zu verbessern. Die Säure ist daher in dem "stromaufwärts" gelegenen Depot enthalten.

Die nanoporösen Materialien besitzen neben der hohen Adsorptionskapazität für polare Wirkstoffe wie Nikotin, auch eine Affinität zu weniger polaren Stoffen, wie z.B. Aromastoffen oder ätherischen Ölen. Derartige Aromastoffe können separat oder simultan zur jeweiligen Substanz, z.B. Nikotin, adsorbiert und zusätzlich freigesetzt werden. Von besonderem Interesse sind in diesem Zusammenhang Terpene und Terpenoide, insbesondere Mono- und Sesquiterpene sowie ätherische Öle oder Feststoffe wie Latschenkieferöl, Eukalyptusöl, Pfefferminzöl, Nelkenöl, Tabakaromaöle oder Menthol.

Damit können Kombinationen von Wirkstoffen mit bevorzugten oder für die jeweilige Anwendung wesentlichen Aromen in den Luftstrom abgegeben werden. Sie können beispielsweise dem angesaugten Luftstrom einen charakteristischen Geschmack verleihen.

Eine oder mehrere weitere Ausführungsformen der Erfindung bestehen darin, verschiedene Wirk-, Aroma- oder Hilfsstoffe, deren Wechselwirkung miteinander zu einem negativen Effekt bei der gemeinsamen Freisetzung führt, in verschiedenen nacheinander geschalteten Hybriddepots unterzubringen, durch welche die Luft strömt.

Die Verfahrensschritte zur Herstellung eines hier beschriebenen Depots für Wirkstoffe und/oder Aromastoffe umfassen gemäß einer oder mehrerer Ausführungsformen die Auswahl einer mikropartikulären Matrix zur Einbettung nanoporösen partikulären Materials, die Auswahl eines nanoporösen partikulären Materials, die gezielte Einstellung der Affinität des nanopartikulären Materials durch Modifikation, die Kosinterung des nanoporösen Materials mit dem Stützmaterial bei einer Temperatur nahe der Schmelz- oder Erweichungstemperatur des als Stützmatrix verwendeten Materials, sowie die Adsorption von Wirk- und/oder Aromastoffen an der Oberfläche der Nanoporen des nanoporösen Materials. Dabei kann eine gezielte Einstellung der adsorbierten Wirkstoffmenge erfolgen.

Als Matrix für die Fixierung nanoporöser Partikel können anorganische Materialien, wie Glas, Silikate oder Alumosilikate oder thermoplastische organische Polymere verwendet werden.

Nach einer oder mehreren Ausführungsformen kann bei der Auswahl des nanoporösen partikulären Materials auch auf den Schritt der gezielten Einstellung der Affinität für die Wirkstoffe oder Aromastoffe verzichtet werden. Wie oben erläutert, können die Verfahrensschritte Kosinterung und Adsorption bzw. Beladung auch miteinander kombiniert werden.

Nanoporöse Materialien aus Siliziumdioxid oder Alumosilikaten werden heutzutage in sehr verschiedenen Ausführungen hergestellt.

Folgende Kriterien können bei der Auswahl für das beschriebene Depot genutzt werden.

### 1. Beladungskapazität

Für ein möglichst geringes Depotvolumen, ist eine hohe Beladbarkeit der nanoporösen Materialien erwünscht. Die Beladungskapazität wird hier prozentual als Masse Wirkstoff (also beispielsweise Masse Nikotin) pro Masse des gefüllten Partikels angegeben. Geeignete Beladungskapazitäten liegen zwischen 20 % und 90%, beispielsweise zwischen 30% bis 85%, insbesondere zwischen 60% und 80%.

### 2. Freisetzbarkeit

In Materialien mit sehr hoher Affinität zum Nikotin oder zu der nikotinhaltigen Verbindung ist der Verteilungskoeffizient zwischen Luft und Oberfläche sehr zu letzterer hin verschoben. Demzufolge ist die Gleichgewichtskonzentration des Nikotins oder der nikotinhaltigen Verbindung in der Luft gering. Insofern sollte die Affinität zum Nikotin oder zu der nikotinhaltigen Verbindung gerade ausreichend sein, um eine effektive Adsorption zu gewährleisten. Für das Einstellen der Wechselwirkungen zwischen Porenoberfläche und Nikotin bzw. nikotinhaltiger Verbindung, die die Affinität bestimmen, wird eine Vorbeschichtung der nanoporösen Partikel mit Polyelektrolyten mit Hilfe der LbL-Technik vorgeschlagen. Je nach Wirkstoff kann der komplementäre Polyelektrolyt so gewählt werden, dass eine kationische, anionische, wasserstoffbrückenbildende oder hydrophobe Oberfläche präsentiert wird.

### 3. Größe der nanoporösen Partikel

Dieser Parameter bestimmt wesentlich das Design des Depotfilters. Ist die Größe der nanoporösen Partikel zu gering, können sie bei ungenügender Fixierung und Filterung mitgerissen werden und aus dem Depot entweichen und beispielsweise in den Atemtrakt des Inhalierenden gelangen. Sind die Partikel zu groß, ist der Austausch der Luft mit dem Wirkstoff, z.B. Nikotin, im Inneren der Partikel unzureichend und die Makroporen des Gerüstes sind verstopft. Geeignet sind beispielsweise Größenverteilungen für die nanoporösen Partikel von 10 µm bis 1 mm, insbesondere von 50 µm bis 700 µm. Besonders geeignet sind irreguläre Partikel einer engen Größenverteilung.

### 4. Porenweite

Die Porenweite der nanoporösen Partikel bestimmt einerseits die verfügbare Oberfläche und somit die Menge des adsorbierten Nikotins, der adsorbierten nikotinhaltigen Verbindung oder des adsorbierten Aromastoffes und andererseits die Geschwindigkeit des Luftaustausches. Während abnehmende Porenweiten zu erhöhter Beladungskapazität, beispielsweise für Nikotin führen, nimmt gleichzeitig der diffusive Luftaustausch ab. Für die Verwendung in einem Depot erwies es sich als vorteilhaft, Materialien mit keiner zu engen Porenverteilung zu verwenden. Porendurchmesser mit einer breiten Verteilung von 1 nm bis 900 nm, insbesondere im Bereich von 3 nm bis 700 nm, sind für eine effektive Adsorption geeignet. Dabei sollte die innere Oberfläche, gemessen durch die BET Stickstoffadsorption, beispielsweise 30-1000 m²/g, vorteilhafterweise 100-600 m²/g betragen.

### 5. Fixierung der nanoporösen Partikel in der makroporösen Matrix

Um ein gleichmäßiges Anströmen der das Nikotin und/oder die nikotinhaltigen Verbindung enthaltenden nanoporösen Partikel mit der durchgeleiteten Luft zu ermöglichen, den Austritt der Partikeln in gegebenenfalls eingeatmete Luft jedoch zu verhindern, sollten sie im Depot stabil fixiert werden. Eine vorteilhafte Ausführungsform eines Depots stellt die Kosinterung der erst bei höheren Temperaturen schmelzenden nanoporösen Partikel mit größeren Sintergranulaten aus Polymeren dar, die einen niedrigeren Schmelz- bzw. Erweichungspunkt besitzen. Der Anteil nanoporöser Partikeln im Sinterstück kann insbesondere zwischen 2% und 40%, beispielsweise zwischen 5% bis 20% liegen. Der Anteil der nanoporösen Partikel sollte dabei so gewählt werden, dass das gesinterte oder angeschmolzene Stützgerüst noch ausreichend mechanisch stabil ist. Beispielsweise können Anteile nanoporöser Partikeln höher als 40% in einigen Fällen nicht mehr die Integrität des makroporösen Stützgerüstes gewährleisten.

Die von den nanoporösen Partikeln auf dem mikroporösen Stützgerüst für die durchströmende Luft bereitgestellte Kontaktfläche zum adsorbiertem Wirkstoff ist im Vergleich zur Kontaktfläche der üblicherweise verwendeten flüssigkeitsgefüllten Kapillaren saugfähiger Materialien sehr groß. Während in letzteren als Verdunstungsfläche lediglich die jeweils zugänglichen Querschnitte (Menisken) der gefüllten Kapillaren in Betracht kommen, vollzieht sich der Gasaustausch im Fall des beschriebenen Depots über dessen gesamte Oberfläche, die den adsorbierten Wirkstoff aufweist.

Untersuchungen zur Adsorption von Stickstoffmolekülen an der Oberfläche von derartigen nanoporösen Materialien gemäß der üblichen BET - Methode zeigen, dass ihre innere Oberfläche mehr als 270 m²/g betragen kann.

Gemäß einer oder mehreren Ausführungsformen werden die nanoporösen Partikel nicht durch Auftropfen des reinen flüssigen Nikotins befüllt. Die Befüllung erfolgt nach Auflösen der Wirkstoffe in einem geeigneten, beispielsweise leicht flüchtigen, organischen Lösungsmittel, das nach vollständigem Verdunsten den Wirkstoff im Depot zurücklässt. Als Lösemittel können beispielsweise Pentan, Hexan, Heptan, Aceton, Ethanol, Methanol oder andere leicht flüchtige organische Lösungsmittel verwendet werden. Nach dem Verdunsten des Lösungsmittels ist der Wirkstoff wie gewünscht an der inneren Oberfläche der nanoporösen Partikel adsorptiv gebunden, ohne den Luftzutritt entscheidend zu behindern. Der Wirkstoff liegt zusätzlich auch an der äußeren Oberfläche der Partikel vor, wobei die innere, von den Nanoporen bereitgestellte Oberfläche wesentlich größer als die äußere Oberfläche der Partikel und des Stützgerüstes ist.

Nanoporöse Materialien mit einer geeigneten Affinität (der Porenoberfläche) zum Wirkstoff (Aromastoff) und die Zugabe einer Wirkstoffmenge (Menge des Aromastoffs) entsprechend der Beladungs- bzw. Adsorptionskapazität des nanoporösen Materials erlauben somit einerseits eine hohe Beladung des Depots mit Nikotin und/oder Aromastoffen und andererseits deren schnelle Freisetzung in einen vorbei- oder durchströmenden Luftstrom. Dabei kann der Gastransport im Inneren der porösen Partikel überwiegend durch Diffusion erfolgen.

Um die Affinität der nanoporösen Materialien sowohl gezielt in Richtung einer optimalen Aufnahme als auch Freisetzung zu modifizieren, wurden verschiedene Möglichkeiten der Vorbehandlung der Porenoberflächen am Beispiel einer vorausgehenden Reinigung, einer Aktivierung, einem Anätzen mit Säuren oder Basen sowie einer Silanisierung untersucht.

Überraschenderweise erwies sich eine einfache Beschichtung der Poren mit Polyelektrolyten nach der Layer-by-Layer- (LbL-)Technik als besonders geeignet, um die Aufnahme bzw. Freisetzung von Wirkstoffen zu steuern. Die LbL-Beschichtung von Nanoporen zum Zweck ihrer Funktionalisierung ist in der Patentanmeldung DE 10 2004 013 637 offenbart. Beispielsweise ist es möglich, durch eine derartige Modifikation mit Polyelektrolyt-Schichten die Freisetzung von Wirkstoffen zu verzögern.

Eine oder mehrere Ausführungsformen nutzen die Temperaturabhängigkeit des Adsorptionsgleichgewichtes der Wirkstoffe bzw. der Aromastoffe an nanoporösen Partikeln. In Abhängigkeit von den Erfordernissen der jeweiligen Anwendung kann dies neben der Einstellung der Affinität der Oberfläche der nanoporösen Partikel für die Wirkstoffe bzw. Aromastoffe über die Temperatur des Depots und/oder über die Temperatur des das Depot durchströmenden Gases oder Gasgemischs selbst geregelt werden.

Vorzugsweise ist im Depot der rauchfreien Zigarette, Zigarre oder Pfeife neben dem Nikotin und/oder der nikotinhaltigen Verbindung mindestens ein Aromastoff gespeichert, der gleichfalls bei Wärmezufuhr freizusetzen ist.

Nachfolgend wird die Erfindung anhand zweier Ausführungsbeispiele unter Bezugnahme auf die Zeichnung näher erläutert. Hierin zeigt:
- Fig. 1: eine Schnittansicht durch ein erstes Ausführungsbeispiel eines erfindungsgemäßen Depots mit Hülse für eine rauchfreie Zigarette,
- Fig. 2: den schematischen Aufbau des Depots nach Fig. 1;
- Fig. 3: eine Aufheizkurve bezogen auf die Zeit bei einem Depot mit Hülse aus Aluminium,
- Fig. 4: einen Temperaturverlauf beim "Rauchen" des Depots bei rauchertypischem Zugverhalten und einer Hülse mit hoher Wärmekapazität;
- Fig. 5: die Freisetzung des Nikotins aus dem Depotfilter in Abhängigkeit von der Temperatur;
- Fig. 6: die Freisetzung von Nikotin aus einem erfindungsgemäßen Depot gemäß Fig. 1 in Abhängigkeit von der Zugzahl (je 35 ml) bei verschiedenen Temperaturen des Filters, und
- Fig. 7: eine Schnittansicht durch ein zweites Ausführungsbeispiel eines erfindungsgemäßen Depots mit Hülse, eingesetzt in eine rauchfreie Zigarette.

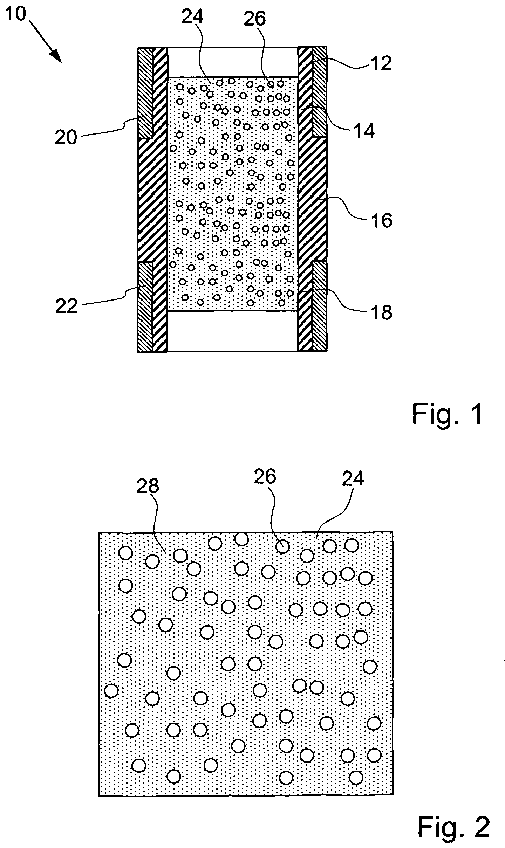

Fig. 1 zeigt in geschnittener Ansicht ein Depot 10 zu Speicherung und Freisetzung von Nikotin für eine nicht näher gezeigte rauchfreie Zigarette. Das Depot 10 hat eine aus Aluminium gefertigte Hülse 12 mit konstantem Innendurchmesser. Die Mantelfläche der Hülse 12 ist gestuft ausgebildet und hat einen ersten Abschnitt 14 kleineren Außendurchmessers, einen als Wärmeübertragungsabschnitt 16 dienenden zweiten Abschnitt größeren Außendurchmessers sowie einen sich an diesen anschließenden dritten Abschnitt 18 kleineren Außendurchmessers. Der erste und der dritte Abschnitt 14 und 18 der Hülse 12 sind jeweils mit einer wärmeisolierenden Papierummantelung 20 und 22 umschlossen und auf der einen Seite mit einem nicht näher gezeigten Mundstück aus einem geeigneten Material versehen ist. Im inneren der Hülse 12 ist ein makroporöses Stützgerüst 24 aufgenommen, in welchem eine Vielzahl nanoporöser Partikel 26 eingebettet ist.

Aus dem durchschnittlichen Zugvolumen von 35 ml und der Zugzeit eines durchschnittlichen Rauchers von 1 bis-2 Sekunden ergibt sich eine sehr kurze Kontaktzeit der das Depot 10 durchströmenden Luft mit dem Nikotinreservoir. Der Übergang des Nikotins in die Gasphase korreliert in etwa mit der Kontaktfläche zwischen dem Nikotinreservoir und der Luft.

Das gezeigte Depot 10 ist in einem Gehäuse der rauchfreien Zigarette so aufgenommen, dass der Wärmeübertragungsabschnitt 16 beim Halten der Zigarette mit der Hand des Benutzers mittelbar oder unmittelbar in Berührung ist. Die Handwärme wird dann an den Wärmeübertragungsabschnitt 16 übertragen, welcher seinerseits die Wärme nach innen an das Stützgerüst 24 weiterleitet. Durch das Erwärmen des Stützgerüstes 24 wird die Freisetzungsrate des Nikotins sowie der Aromastoffe erhöht, so dass bei jedem Zug eine ausreichende Menge Nikotin und Aromastoffe vom Benutzer konsumiert werden kann. Die beiden Papierummantelungen 20 und 22 verhindern dabei eine Wärmeabstrahlung nach außen, so dass das Stützgerüst 24 gleichmäßig erwärmt bleibt.

Das Volumen der Hülse 12 gemäß Fig. 1 wird über deren Wandstärke so angepasst, dass die Aufwärmzeit nach der Positionierung zwischen den Fingern möglichst schnell verläuft.

Fig. 2 zeigt den schematischen Aufbau des Stützgerüstes 24 des Depots 10 zur Speicherung von Nikotin und Aromstoffen. Das Stützgerüst 24 ist ein aus gesinterten Partikeln gebildetes makroporöses Stützgerüst 24. In dem Stützgerüst 24 sind Poren, Kavernen und Zwischenräume 28 ausgebildet, die nach dem Sintern erhalten geblieben sind. Die Kavernen und Zwischenräume 28 sind dabei so ausgebildet, dass das Stützgerüst 24 einen definierten Zugwiderstand für einen von einem Benutzer anzusaugenden Luftstrom aufweist.

Die Poren, Kavernen und Zwischenräume 28 bilden ein durchgängiges Netzwerk in dem die nanoporösen Partikel 26 stabil und dauerhaft angehaftet bzw. eingebettet sind. Die nanoporösen Partikel 26 weisen Poren im Bereich von 1 nm bis 900 nm auf. An der Oberfläche der nanoporösen Partikel 26, insbesondere auf deren inneren, von den Porenwänden gebildeten Oberflächen sind das Nikotin und gegebenenfalls nikotinhaltige Verbindungen sowie die Aromastoffe adsorbiert und stehen beim Ansaugen von Luft durch das Depot 10 im ständigen Austausch mit der durch das Stützgerüst 24 strömenden Luft.

Das hier beschriebene Depot 10 kann einerseits eine ausreichende NikotinFreisetzung in die Atemluft gewährleisten und andererseits das Nikotin vor Oxidation schützen.

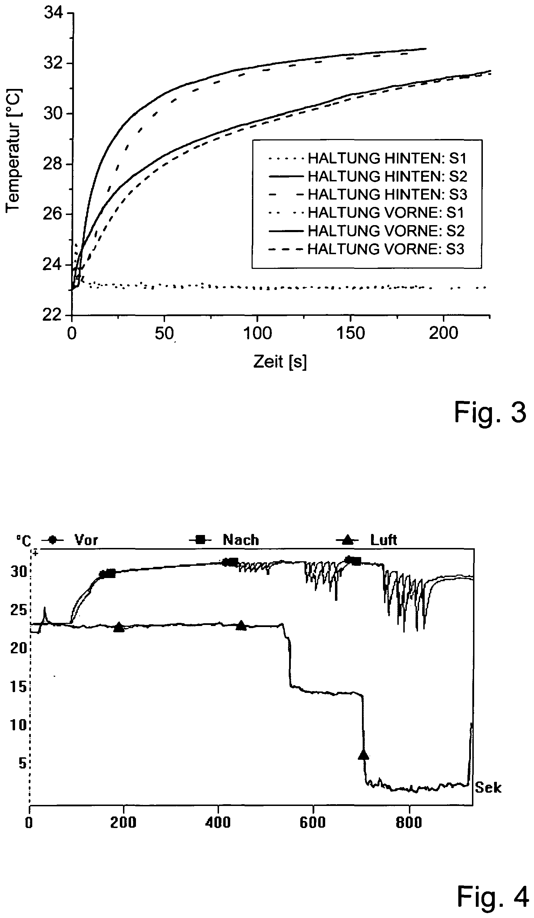

Als Beispiel ist in Fig. 3 der Temperaturverlauf eines Depots 10 mit einer Hülse 12 aus Aluminium mit einer Wandstärke von 1 mm (oben/unten) bzw. 1.5 mm an der Fingerkontaktfläche und des Depots nach dem Aufnehmen und Einschluß in die Finger bei 23°C Lufttemperatur gezeigt. Dabei ist die Position der Hülse 12 zwischen den Fingern vorzugsweise so, dass die Kontaktfläche bei einer Haltung zwischen den zweiten Fingergliedknochen gesehen von der Fingerspitze (Haltung vorne) zwischen Zeige- und Mittelfinger geringer ist als zwischen dem dritten Fingergliedknochen (Haltung hinten). Mit abnehmendem Volumen der Hülse 12 bzw. geringerer Wärmekapazität verläuft die Erwärmung ebenfalls schneller. Somit sollte die Wandstärke der Hülse 12 vorteilhafterweise zwischen 0.2 und 2.5 mm, ganz besonders bevorzugt sind 0.4 bis 1.5 mm.

Fig. 3 zeigt dabei Aufheizkurven der Hülse 12 aus Aluminium (Wandstärke 1/1.5 mm) durch die Finger bei Haltung zwischen Zeige- und Mittelfinger. Dabei bedeuteten im Diagramm die Angabe "Haltung vorn" ein Halten der Hülse 12 zwischen zweitem Fingerknochen von der Fingerspitze gesehen, "Haltung hinten" ein Halten der Hülse 12 zwischen dem dritten Fingerknochen, "S1" die Umgebungstemperatur, "S2" die Temperatur der Hülse 12 sowie "S3" die Temperatur in der Mitte des Depots.

Die Wärmekapazität der erwärmten Hülse 12 muss wiederum ausreichen, um beim kräftigen Ziehen in kalter Luft die Abkühlung des Filters durch die hindurchströmende Luftmenge von 35 bis 50 ml möglichst rasch zu kompensieren. Dazu wurden Messungen der Hülsen- und Filtertemperatur am Ausgang des Depots 10 bei verschiedenen Lufttemperaturen durchgeführt.

Fig. 4 zeigt den Temperaturverlauf beim "Rauchen" des Depots 10 bei rauchertypischem Zugverhalten und einer Hülse 12 mit hoher Wärmekapazität. Es ist klar zu sehen, dass die Temperatur am Filterdepot stärker einbricht als an der Hülse, sie sich jedoch wieder recht schnell aufgrund der Wärmenachlieferung durch die Hülse 12 erholt. Selbst bei einer Lufttemperatur von nur 3°C sinkt die Temperatur am Ausgang des Depots 10 nicht unter 23°C. Bei Hülsen 12 mit geringerer Wärmekapazität werden die Temperatureinbrüche stärker und die Erholungszeiten bis zum Erreichen der Ausgangstemperatur speziell bei kalter Außenluft länger.

Die in Fig. 4 gezeigte Temperaturfunktionen entsprechen der Temperatur der Umgebung (Luft), der Wärmeübergangshülse 12 vor dem Lufteintritt sowie des Depots 10 nach der Mitte am Luftaustritt bei typischer Inhalation eines Rauchers von Luft unterschiedlicher Temperaturen.

In der nachfolgenden Tabelle sind die Messwerte der Gesamtnikotinfreisetzung beim Rauchen einer kalten Zigarette und der Fingerzigarette (bei 24°C Lufttemperatur) und der Faktor der Verbesserung durch die erfindungsgemäße Zigarette aufgelistet:

| Filtertemperatur in °C | Nikotinausbeute bei 15 Zügen a 35 ml nach DIN... | Faktor der Nikotinausbeuteerhöhung bei Fingererwärmung gegenüber einer kalten Zigarette |
|---|---|---|
| 32 | 0.25 | 1 |
| 24 | 0.13 | 1.9 |
| 14 | 0.056 | 4.5 |
| 5 | 0.026 | 9.6 |

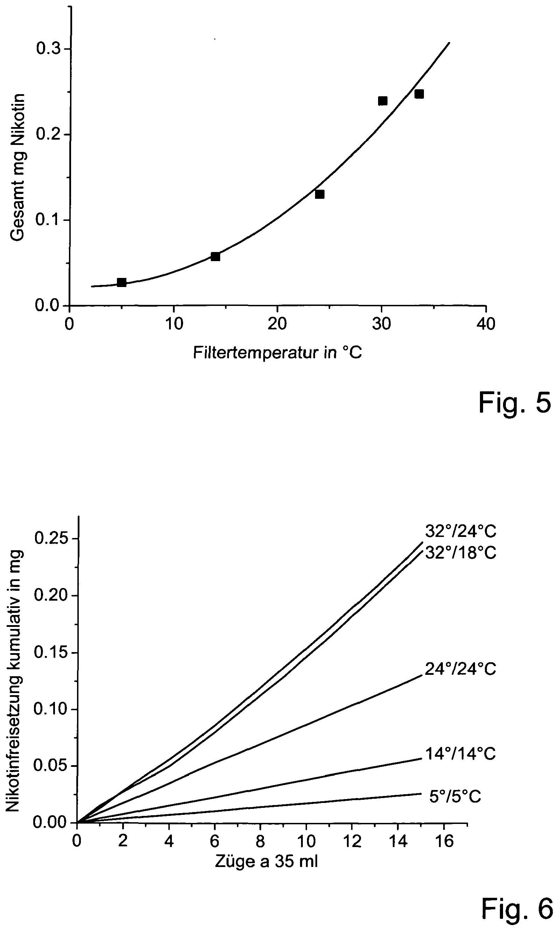

In Fig. 5 ist die Freisetzung des Nikotins aus dem Depot 10 in Abhängigkeit von der Temperatur des Depots gezeigt, während Fig. 6 die Freisetzung von Nikotin aus dem Depot 10 in Abhängigkeit von der Zugzahl (je 35 ml) bei verschiedenen Temperaturen des Depots 10 (linke Zahl an den Kurven) und der angesaugten Luft (rechte Zahl) zeigen. Wie zu erkennen ist, wird die Nikotinfreisetzung bei der Fingerzigarette kaum von der Umgebungstemperatur beeinflusst, wogegen sie bei einer Zigarette ohne Wärmeübergangsfläche mit sinkender Temperatur stark abnimmt.

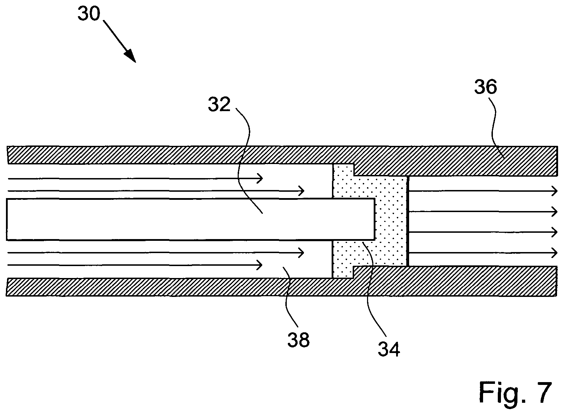

Fig. 7 zeigt ein zweites Ausführungsbeispiel eines Depots 30 mit einer Heizquelle 32 die über eine externe Energiezufuhr verfügt.

Bei diesem zweiten Ausführungsbeispiel wird die Heizquelle 32 so angeordnete, dass sie zur Wärmeübertragung mit einem Wärmeübertragungsabschnitt 34 im Inneren des Depots 30 zusammenwirkt (Kommentar: in der Zeichnung ist der Pfeil nicht exakt auf das Depot gerichtet). Die Hülse 36 ist hierbei aus einem Material mit hoher Wärmeisolation gefertigt, während der das Depots 30 entsprechend der zuvor geschilderten Weise aufgebaut ist. In einer Ausbuchtung in dem Depot 30 ist paßgenau die Heizquelle 32 eingeführt, welche in Form eines Stabes mit hoher Wärmeleitfähigkeit Wärme auf das Depot 30 überträgt. Die angesaugte, bereits leicht vorgewärmte Luft 38 passiert das aufgeheizte Depot 30 und verlässt, weitgehend mit Nikotin gesättigt das Depot 30 in Richtung Mund.

### Bezugszeichenliste:

- 10: Depot
- 12: Hülse mit hoher Wärmeleitfähigkeit
- 14: erste Abschnitt kleineren Außendurchmessers
- 16: Wärmeübertragungsabschnitt
- 18: dritter Abschnitt kleineren Außendurchmessers
- 20: Papierummantelung
- 22: Papierummantelung mit Mundstück
- 24: makroporöses Stützgerüst
- 26: nanoporöse Partikel
- 28: Kavernen und Zwischenräume

- 30: Depot
- 32: Heizquelle
- 34: Wärmeübertragungsabschnitt
- 36: isolierende Hülse
- 38: vorgewärmte Luft

## Patentansprüche

1. Rauchfreie Zigarette, Zigarre oder Pfeife mit mindestens einem Depot (10) für die Speicherung und definierte, durch externe Wärmezufuhr bewirkte Freisetzung von Nikotin und/oder mindestens einer nikotinhaltigen Verbindung an einen durch das Depot (10) zu leitenden Luftstrom, wobei das Depot (10) mindestens einen Wärmeübertragungsabschnitt (16; 34) für eine gezielte Wärmezufuhr zur definierten Freisetzung des Nikotins und/oder der Nikotinverbindung an den Luftstrom aufweist, **dadurch gekennzeichnet, dass** das Depot (10) zum Speichern des Nikotins und/oder der Nikotinverbindung und zum Freisetzen ausreichender Mengen des Nikotins und/oder der Nikotinverbindung an den Luftstrom eine Hybridstruktur aus einem offenporigen makroporösen Stützgerüst (24), durch das der Luftstrom strömen kann, und im Stützgerüst (24) vorgesehenen Nanoporen (26) zur Speicherung und Freisetzung des Nikotins und/oder der nikotinhaltigen Verbindung aufweist, wobei das Stützgerüst (24) aus dauerhaft miteinander verbundenen Partikeln (28) oder Fasern besteht und die Nanoporen in nanoporösen Partikeln (26) ausgebildet sind, welche im Stützgerüst (24) immobilisiert gehalten sind, wobei die Wärmeleitfähigkeit des Materials für den Wärmeübertragungsabschnitt in einem Bereich von 30 bis 450 W/(m·K) liegt.

2. Rauchfreie Zigarette, Zigarre oder Pfeife nach Anspruch 1, **dadurch gekennzeichnet, dass** das Depot (10) eine das Depot (10) ummantelnde Hülse (12) aufweist, welche für eine gezielte Wärmezufuhr zumindest abschnittsweise aus dem Material mit hoher Wärmeleitfähigkeit zur Bildung des Wärmeübertragungsabschnittes gefertigt ist.

3. Rauchfreie Zigarette, Zigarre oder Pfeife nach Anspruch 1 und 2, **dadurch gekennzeichnet, dass** die Länge der ummantelnden Hülse (12) so dimensioniert ist, dass ein für die Freisetzung des Nikotins oder der nikotinhaltigen Verbindung ausreichender Wärmeübertragung zwischen zwei Fingern einer Hand, vorzugsweise zwischen den zweiten oder dritten Fingergliedern, an das Depot erfolgen kann.

4. Rauchfreie Zigarette, Zigarre oder Pfeife nach Anspruch 1, 2 oder 3, **dadurch gekennzeichnet, dass** die ummantelnde Hülse (12) eine Wärmekapazität aufweist, die einerseits ausreicht, um das Depot (10) auch beim Durchsaugen kalter Luft schnell wieder auf die Fingertemperatur zu bringen und andererseits so gering ist, dass die Aufheizzeit durch die Finger möglichst schnell erfolgt.

5. Rauchfreie Zigarette, Zigarre oder Pfeife nach Anspruch 1, **dadurch gekennzeichnet, dass** das Depot (30) im Inneren mit einer Heizquelle (32) koppelbar und für eine gezielte Wärmezufuhr über denWärmeübergangsabschnitt (34) zumindest abschnittsweise aus einem Material mit hoher Wärmeleitfähigkeit gefertigt ist, wobei die Wärmeleitfähigkeit des Materials für den Wärmeübergangsabschnitt in einem Bereich von 30 bis 450 W/(m·K).

6. Rauchfreie Zigarette, Zigarre oder Pfeife nach Anspruch 5, **dadurch gekennzeichnet, dass** das beheizte Depot (30) im Inneren mit einer wärmeisolierenden Hülse (36) umgeben ist, die Wärmeverluste minimiert und die Hand vor Verbrennungen schützt.

7. Rauchfreie Zigarette, Zigarre oder Pfeife nach einem der vorhergehenden Ansprüche, **dadurch gekennzeichnet, dass** die Wärmeleitfähigkeit des Materials für die Wärmeübergangsfläche in einem Bereich von 85 bis 300 W/(m·K), vorzugsweise in einem Bereich von 185 bis 250 W/(m·K) liegt.

8. Rauchfreie Zigarette, Zigarre oder Pfeife nach einem der vorhergehenden Ansprüche, **dadurch gekennzeichnet, dass** die nanoporösen Partikel (26) aus einem anderen Material als das Stützgerüst (24) bestehen.

9. Rauchfreie Zigarette, Zigarre oder Pfeife nach einem der vorhergehenden Ansprüche, **dadurch gekennzeichnet, dass** das Stützgerüst (24) im Wesentlichen aus einem anorganischen Material, wie Glas, Silikaten oder Alumosilikaten, besteht und die Nanoporen im Material des Stützgerüstes ausgebildet sind.

10. Rauchfreie Zigarette, Zigarre oder Pfeife nach einem der vorhergehenden Ansprüche, **dadurch gekennzeichnet, dass** im Depot (10; 30) neben dem Nikotin und/oder der nikotinhaltigen Verbindung mindestens ein Aromastoff gespeichert ist, welcher bei Wärmezufuhr freizusetzen ist.

11. Rauchfreie Zigarette, Zigarre oder Pfeife nach einem der vorhergehenden Ansprüche, **dadurch gekennzeichnet, dass** die Zigarette, Zigarre oder Pfeife einen Grundkörper aufweist, in welchem das als Einwegartikel ausgebildet Depot (10; 30) aufgenommen ist.

## Claims

1. A smokeless cigarette, cigar, or pipe having at least one depot (10) for storage and defined release by external application of heat of nicotine and/or at least one compound comprising nicotine induced into an airstream flowing through the depot (10), the depot (10) comprising at least one heat transfer segment (16; 34) for targeted heat input for defined release of the nicotine and/or nicotine compound to the airflow, **characterized in that** the depot (10) comprises a hybrid structure for storing the nicotine and/or the nicotine compound and for releasing sufficient amounts of nicotine and/or nicotine compound into the airflow, and having an open-pored macroporous support matrix (24) through which the airflow can flow, and having nanopores (26) in the support matrix (24) for storing and releasing the nicotine and/or the nicotine compound, wherein the support matrix (24) is made from particles (28) or fibers permanently bonded to each other and the nanopores are implemented in nanoporous particles (26) held immobile in the support matrix (24), wherein the thermal conductivity of the material for the heat transfer segment is in a range from 30 to 450 W/(m-K).

2. The smokeless cigarette, cigar, or pipe according to claim 1, **characterized in that** the depot (10) comprises a sleeve (12) surrounding the depot (10) and produced at least in segments from the material having higher thermal conductivity for forming the heat transfer segment for targeted heat input.

3. The smokeless cigarette, cigar, or pipe according to claim 1 and 2, **characterized in that** the length of the enclosing sleeve (12) is dimensioned so that heat transfer to the depot can take place between two fingers of one hand, preferably between the second or third phalanges, in sufficient quantity for releasing the nicotine or compound comprising nicotine.

4. The smokeless cigarette, cigar, or pipe according to claim 1, 2, or 3, **characterized in that** the enclosing sleeve (12) comprises a heat capacity that is high enough for bringing the depot (10) back to finger temperature quickly when drawing in cold air, and low enough so that the time for heating by the fingers is as short as possible.

5. The smokeless cigarette, cigar, or pipe according to claim 1, **characterized in that** the interior of the depot (30) can be coupled to a heating source (32) and is made from a material having higher thermal conductivity at least in segments for targeted heat input via the heat transfer surface (34), wherein the thermal conductivity of the material for the heat transfer segment is in a range from 30 to 450 W/(m-K).

6. The smokeless cigarette, cigar, or pipe according to claim 5, **characterized in that** the interior of the heated depot (30) is surrounded by a thermally insulated sleeve (36) for minimizing heat losses and protecting the hand from burns.

7. The smokeless cigarette, cigar, or pipe according to any one of the preceding claims, **characterized in that** the thermal conductivity of the material for the heat transfer surface is in a range from 85 to 300 W/(m·K), preferably in a range from 185 to 250 W/(m·K).

8. The smokeless cigarette, cigar, or pipe according to any one of the preceding claims, **characterized in that** the nanoporous particles (26) are made of a different material than the support matrix (24).

9. The smokeless cigarette, cigar, or pipe according to any one of the preceding claims, **characterized in that** the support matrix (24) is substantially made of an inorganic material such as glass, silicates, or alumosilicates, and the nanopores are implemented in the material of the support matrix.

10. The smokeless cigarette, cigar, or pipe according to any one of the preceding claims, **characterized in that** at least one flavoring agent is stored in the depot (10; 30) in addition to the nicotine and/or nicotine compound and is released when heat is input.

11. The smokeless cigarette, cigar, or pipe according to any one of the preceding claims, **characterized in that** the cigarette, cigar, or pipe comprises a base body in which the depot (10; 30) implemented as a disposable item is received.

## Revendications

1. Cigarette, cigare ou pipe sans fumée, comprenant au moins un dépôt (10) pour le stockage et la libération définie de nicotine et/ou d'au moins un dérivé contenant de la nicotine, provoquée par un apport externe de chaleur, dans un courant d'air devant être conduit à travers le dépôt (10), le dépôt (10) présentant au moins un tronçon de transfert de chaleur (16 ; 34) pour un apport de chaleur ciblé en vue de la libération définie de nicotine et/ou du dérivé contenant de la nicotine dans le courant d'air, caractérisé(e) en ce que le dépôt (10) pour le stockage de la nicotine et/ou du dérivé contenant de la nicotine et pour la libération de quantités suffisantes de nicotine et/ou du dérivé contenant de la nicotine dans le courant d'air présente une structure hybride constituée d'une architecture support (24) macroporeuse à pores ouverts, à travers laquelle le courant d'air peut circuler, et de nanopores (26) aménagés dans l'architecture support (24) pour le stockage et la libération de la nicotine et/ou du dérivé contenant de la nicotine, l'architecture support (24) se composant de particules (28) ou de fibres liées durablement les unes aux autres et les nanopores étant formés dans des particules (26) nanoporeuses qui sont maintenues immobilisées dans l'architecture support (24), la conductivité thermique du matériau pour le tronçon de transfert de chaleur se trouvant dans une plage de 30 à 450 W/(mK).

2. Cigarette, cigare ou pipe sans fumée selon la revendication 1, caractérisé(e) en ce que le dépôt (10) présente une gaine (12) enveloppant le dépôt (10), qui, pour un apport de chaleur ciblé, est confectionnée au moins par tronçons à partir du matériau ayant une conductivité thermique élevée aux fins de la formation du tronçon de transfert de chaleur.

3. Cigarette, cigare ou pipe sans fumée selon la revendication 1 et 2, caractérisé(e) en ce que la longueur de la gaine (12) d'enveloppement est dimensionnée, de sorte qu'un transfert de chaleur suffisant pour la libération de la nicotine ou du dérivé contenant de la nicotine puisse se faire entre les deux doigts d'une main, de préférence entre les deuxièmes ou troisièmes doigts, en direction du dépôt.

4. Cigarette, cigare ou pipe sans fumée selon la revendication 1, 2 ou 3, caractérisé(e) en ce que la gaine (12) d'enveloppement présente une capacité calorifique qui suffit d'une part pour porter à nouveau rapidement le dépôt (10) à la température des doigts même en cas d'aspiration d'air froid et qui est d'autre part suffisamment faible pour que le temps de chauffage par les doigts soit le plus rapide possible.

5. Cigarette, cigare ou pipe sans fumée selon la revendication 1, caractérisé(e) en ce que le dépôt (30) peut être couplé en interne avec une source de chaleur (32) et pour un apport de chaleur ciblé par le biais du tronçon de transfert de chaleur (34), est confectionné au moins par tronçons à partir d'un matériau ayant une conductivité thermique élevée, la conductivité thermique du matériau pour le tronçon de transfert de chaleur étant située dans une plage de 30 à 450 W/(mK).

6. Cigarette, cigare ou pipe sans fumée selon la revendication 5, caractérisé(e) en ce que le dépôt (30) chauffé est enveloppé en interne d'une gaine (36) thermo-isolante, qui réduit les pertes de chaleur et protège la main des brûlures.

7. Cigarette, cigare ou pipe sans fumée selon l'une des revendications précédentes, caractérisé(e) en ce que la conductivité thermique du matériau pour la surface de transfert de chaleur est située dans une plage de 85 à 300 W/(mK), de préférence dans une plage de 185 à 250 W/(mK).

8. Cigarette, cigare ou pipe sans fumée selon l'une des revendications précédentes, caractérisé(e) en ce que les particules (26) nanoporeuses se composent d'un autre matériau que l'architecture support (24).

9. Cigarette, cigare ou pipe sans fumée selon l'une des revendications précédentes, caractérisé(e) en ce que l'architecture support (24) se compose essentiellement d'un matériau inorganique, tel que le verre, les silicates ou les aluminosilicates et que les nanopores sont formés dans le matériau de l'architecture support.

10. Cigarette, cigare ou pipe sans fumée selon l'une des revendications précédentes, caractérisé(e) en ce qu'au moins une substance aromatique devant être libérée lors de l'apport de chaleur est stockée dans le dépôt (10 ; 30) en plus de la nicotine et/ou du dérivé contenant de la nicotine.

11. Cigarette, cigare ou pipe sans fumée selon l'une des revendications précédentes, caractérisé(e) en ce que la cigarette, le cigare ou la pipe présente un corps de base dans lequel est réceptionné le dépôt (10 ; 30) conçu sous forme d'article à usage unique.
